# EUROPEAN PATENT APPLICATION

(11) **EP 1 375 534 A1**
(43) Date of publication of application: **02.01.2004**
(21) Application number: 02715864.1
(22) Date of filing: 22.01.2002
(51) Int. Cl.: C08F 8/00, B01J 31/22, B01J 31/28, C07C 51/00, C07C 63/06, C07C 67/28, C07C 69/78, C07C 51/23, C07C 53/126, C07C 49/223, C07C 49/78, C07C 49/413, C07C 47/54, C07C 45/38, C07C 45/39, C07C 45/36, C07C 45/68, C07C 1/32, C07C 15/14

(54) **SOLID-PHASE-SUPPORTED TRANSITION METAL CATALYSTS**

(30) Priority: 12.03.2001 JP 2001068333
(71) Applicant: Zeria Pharmaceutical Co., Ltd., Tokyo 103-8351 (JP)
(72) Inventor: UOZUMI, Yasuhiro, NAGOYA-SHI, Aichi 458-0003 (JP); NAKAO, Ryu, ZERIA PHARMACEUTICAL CO. LTD C. R. L., OSATO-GUN, Saitama 360-0111 (JP)
(74) Representative: Hartz, Nikolai, Dr.
(86) International application number: PCT/JP2002/000414
(87) International publication number: WO 2002/072644

(57) **Abstract**

Solid-supported transition metal complex catalysts each of which is represented by the following formula (I) : 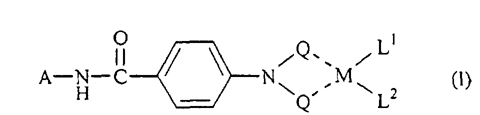 wherein A represents a polystyrene-polyethylene glycol copolymer resin, Q represents a heterocycle which may be substituted by one or more lower alkyl groups, lower alkoxy groups or halogen atoms, L¹ and L² may be the same or different and each represents a halogen atom or an acetoxy, trifluoroacetoxy, trifluoromethanesulfonyl, tetrafluoroborate or π-allyl group, and M represents copper, palladium, nickel, cobalt, rhodium or platinum; and S-solid-supported transition metal catalysts each of which comprises a compound, which is represented by the following formula (II): 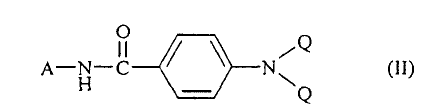 wherein A and Q have the same meanings as defined above, and a transition metal selected from copper, palladium, nickel, cobalt, rhodium or platinum and supported on said compound.

These catalysts are usable for a wide variety of reactions, permit conducting the reactions in water systems, exhibit sufficient catalytic functions even in oxygen atmosphere, and allow recovery and reuse.

## Description

### Technical Field

This invention relates to reaction catalysts, and specifically, to amphiphilic, solid-supported transition metal catalysts.

### Background Art

In the syntheses of organic compounds, metal catalysts such as copper, palladium, nickel, cobalt, rhodium and platinum are used for various reactions such as oxidation of alcohols, allylic oxidation, and formation of carbon-carbon bonds, and in such reactions, organic solvents are also used. Further, amphiphilic, solid-supported phosphine-palladium complex catalysts, which are metal complex catalysts supported on solid phases and having amphiphilic properties, are known to be usable in CO insertion reactions, Suzuki-Miyaura reactions and allylic substitution reactions (Tetrahedron Letters, **38**, 3557-3560 (1997); Tetrahedron Letters, **39**, 8303-8306 (1998); J. Org. Chem., **64**, 3384-3388 (1999); J. Org. Chem., **64**, 6921-6923 (1999)).

When a metal catalyst is used, an organic solvent is also used to heighten the reactivity so that potential risks such as flammability, toxicity to organisms and environmental pollution should be taken. The use of such an organic solvent is also accompanied by an inconvenience in handling that attention should be also paid to filtration, recovery, disposal and the like of the catalyst after use. Further, amphiphilic, solid-supported phosphine-palladium complex catalysts show high catalytic activities in water, but involve a problem in that under oxygen conditions such as oxygen atmosphere, they do not exhibit functions as catalysts because phosphine is oxidized.

Desired for the syntheses of organic compounds is a catalyst free of the above-described problems, that is, a catalyst which exhibits its effects without using an organic solvent from the standpoint of environmental pollution, which exhibits its catalytic functions sufficiently even under oxygen atmosphere, which can be used for a wide variety of reactions, and which can be recovered with ease and can be reused.

Such a catalyst has a merit that it can be used for combinatorial chemistry synthesis which is actively practiced in recent years.

### Disclosure of the Invention

Under such circumstances, the present inventors have proceeded with extensive research. As a result, it has been found that a solid-supported transition metal complex catalyst represented by the below-described formula (I) and a solid-supported transition metal catalyst comprising a compound represented by the formula (II) and a particular transition metal supported on the compound can be used for a wide variety of reactions, exhibit sufficient catalytic functions even in a water system or under oxygen atmosphere, and moreover, can be recovered and reused, leading to the completion of the present invention.

Described specifically, the present invention provides a solid-supported transition metal complex catalyst represented by the following formula (I): 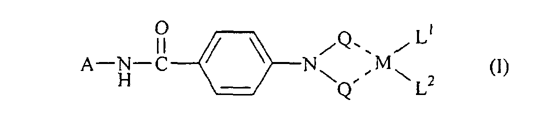 wherein A represents a polystyrene-polyethylene glycol copolymer resin, Q represents a heterocycle which may be substituted by one or more lower alkyl groups, lower alkoxy groups or halogen atoms, L¹ and L² may be the same or different and each represents a halogen atom or an acetoxy group, trifluoroacetoxy group, trifluoromethanesulfonyl group, tetrafluoroborate group or π-allyl group, and M represents copper, palladium, nickel, cobalt, rhodium or platinum.

The present invention also provides a solid-supported transition metal catalyst comprising a compound, which is represented by the following formula (II): 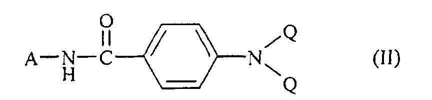 wherein A and Q have the same meanings as defined above, and a transition metal selected from copper, palladium, nickel, cobalt, rhodium or platinum and supported on the compound.

Further, the present invention also provides a compound, which is represented by the following formula (II) : 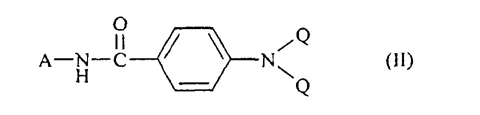 wherein A and Q have the same meanings as defined above.

### Brief Description of the Drawings

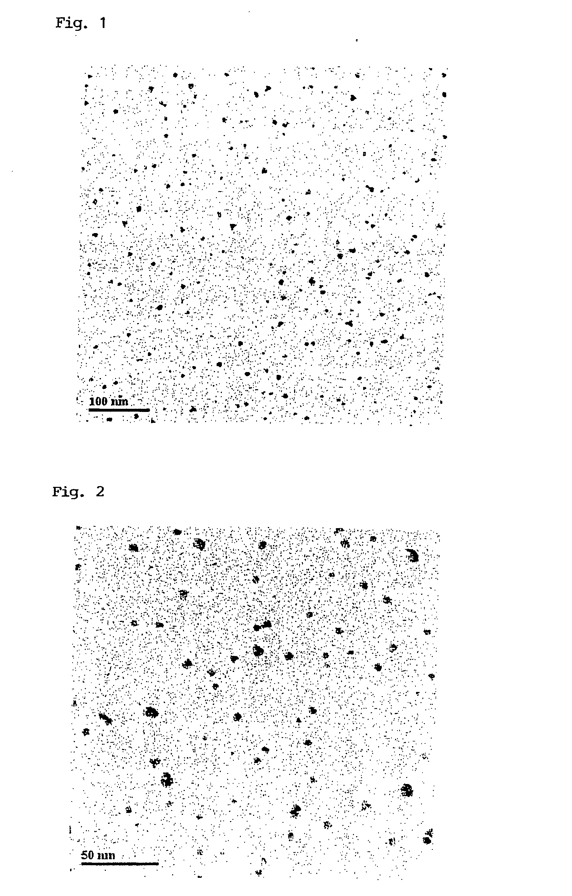

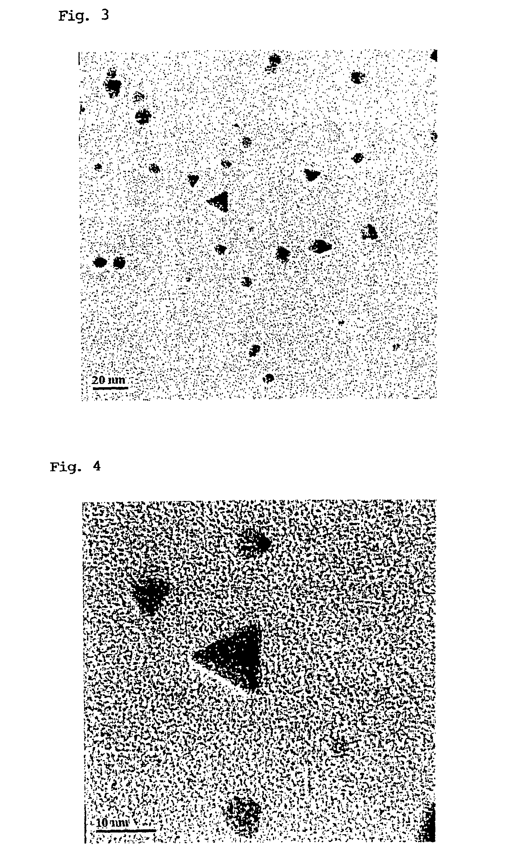

FIGS. 1 to 4 show electron micrographs of a solid-supported palladium catalyst obtained in Example 5.

### Best Modes for Carrying out the Invention

The term "lower" as used herein means a linear, branched or cyclic carbon chain having 1 to 6 carbon atoms.

Accordingly, the term "lower alkyl group" can mean a linear, branched or cyclic alkyl groups having 1 to 6 carbon atoms, including, for example, methyl group, ethyl group, propyl group, isopropyl group, cyclopropyl group, butyl group, isobutyl group, sec-butyl group, tert-butyl group, cyclobutyl group, pentyl group, 1-methylbutyl group, 2-methylbutyl group, isopentyl group, tert-pentyl group, 1,2-dimethylpropyl group, neopentyl group, 1-ethylpropyl group, cyclopentyl group, hexyl group, 1-methylpentyl group, 2-methylpentyl group, 3-methylpentyl group, isohexyl group, 1-ethylbutyl group, 2-ethylbutyl group, 1,1-dimethylbutyl group, 1,2-dimethylbutyl group, 1,3-dimethylbutyl group, 2,2-dimethylbutyl group, 2,3-dimethylbutyl group, 3,3-dimethylbutyl group, 1-methyl-1-ethylpropyl group, 1-ethyl-2-methylpropyl group, 1,1,2-trimethylpropyl group, 1,2,2-trimethylpropyl group, and cyclohexyl group.

The term "lower alkoxy group", on the other hand, can mean a linear, branched or cyclic alkoxy group having 1 to 6 carbon atoms, including, for example, methoxy group, ethoxy group, propoxy group, isopropoxy group, cyclopropoxy group, butoxy group, isobutoxy group, sec-butoxy group, tert-butoxy group, cyclobutoxy group, pentyloxy group, 1-methylbutoxy group, 2-methylbutoxy group, isopentyloxy group, tert-pentyloxy group, 1,2-dimethylpropoxy group, neopentyloxy group, 1-ethylpropoxy group, cyclopentyloxy group, hexyloxy group, 1-methylpentyloxy group, 2-methylpentyloxy group, 3-methylpentyloxy group, isohexyloxy group, 1-ethylbutoxy group, 2-ethylbutoxy group, 1,1-dimethylbutoxy group, 1,2-dimethylbutoxy group, 1,3-dimethylbutoxy group, 2,2-dimethylbutoxy group, 2,3-dimethylbutoxy group, 3,3-dimethylbutoxy group, 1-methyl-1-ethylpropoxy group, 1-ethyl-2-methylpropoxy group, 1,1,2-trimethylpropoxy group, 1,2,2-trimethylpropoxy group, and cyclohexyloxy group.

Further, the term "halogen atom" can mean a fluorine, chlorine, bromine or iodine atom.

In the present invention, the polystyrene-polyethylene glycol copolymer resin represented by A can preferably be one having an amino group at an end thereof, that is, one having the structure of A-NH₂. Illustrative are those having the following structural formula: 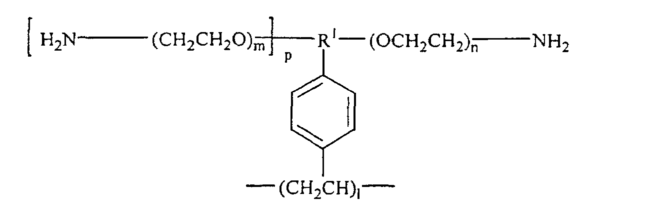 wherein R¹ represents a crosslinking group, and p stands for 0 or 1.

Here, the crosslinking group can preferably be a linear or branched alkylene group, with one having 1 to 12 carbon atoms being particularly preferred.

Usable examples of such a resin can include "ArgoGel" (trade mark), "TentaGel" (trade mark) and "NovaGel" (trade mark), with "ArgoGel" having amino groups at ends thereof being particularly preferred.

"ArgoGel" which has amino groups at ends thereof has the following structure:

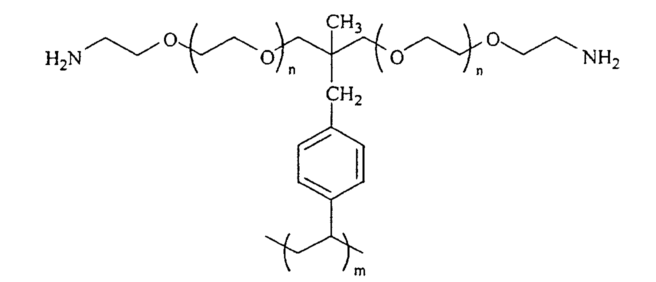

Further, L¹ and L² may be the same or different and each represents a halogen atom or an acetoxy group, trifluoroacetoxy group, trifluoromethanesulfonyl group, tetrafluoroborate group or π-allyl group, with a halogen atom or an acetoxy group, trifluoroacetoxy group or π-allyl group being preferred. It is particularly preferred that L¹ and L² are the same and each represents an acetoxy group or trifluoroacetoxy group or one of L¹ and L² is a π-allyl group and the other is a halogen atom.

M is a transition metal which is copper, palladium, nickel, cobalt, rhodium or platinum. As the transition metal for use in the present invention, copper or palladium is particularly preferred.

Q is a heterocycle which may be substituted by one or more lower alkyl groups, lower alkoxy groups or halogen atoms. The heterocycle can mean a five-membered or six-membered ring having one or more nitrogen, sulfur and/or oxygen atoms. Examples can include pyrrole, imidazole, imidazoline, triazole, tetrazole, pyrazole, pyridine, pyrazine, pyrimidine, pyridazine, oxazoline, oxazole, isoxazole, thiazoline, thiazole, isothiazole, and thiophene. Among these, pyridine is preferred.

The solid-supported transition metal complex catalyst (I) according to the present invention can be produced, for example, in accordance with the following reaction scheme 1 and reaction scheme 2. 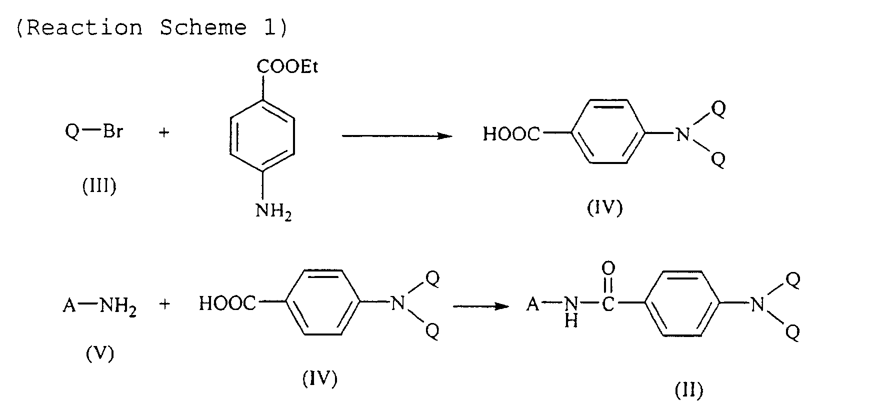 wherein A and Q have the same meanings as defined above.

Specifically, a brominated heterocycle (III) and ethyl p-aminobenzoate are reacted under argon atmosphere in the presence of tris(dibenzylideneacetone)dipalladium, rac-2,2'-bis(diphenylphosphino)-1,1'-binaphthyl and a metal alcoholate to yield a benzoic acid derivative (IV). The brominated heterocycle (III) is required in an amount at least twice as much as ethyl p-aminobenzoate, and the metal alcoholate can preferably be a sodium alcoholate with sodium tert-butoxide being particularly preferred. A reaction solvent can be an organic solvent such as benzene, toluene, xylene, acetonitrile or acetone, and depending on the reaction temperature, one or more of such organic solvents can be chosen and used as needed.

To a Merrifield vessel purged with nitrogen gas, a polystyrene-polyethylene glycol copolymer resin (V) having an amino group at an end thereof is then added, followed by the addition of the benzoic acid derivative (IV), 1-ethyl-3-(3-dimethylaminopropyl)carbodiimide hydrochloride and 1-hydroxybenzotriazole. The resulting mixture is then shaken in dimethylformamide for 10 to 30 hours to afford a compound (II).

This compound (II) is useful as an intermediate for the production of the solid-supported transition metal complex catalyst (I) and the solid-supported transition metal catalyst according to the present invention. 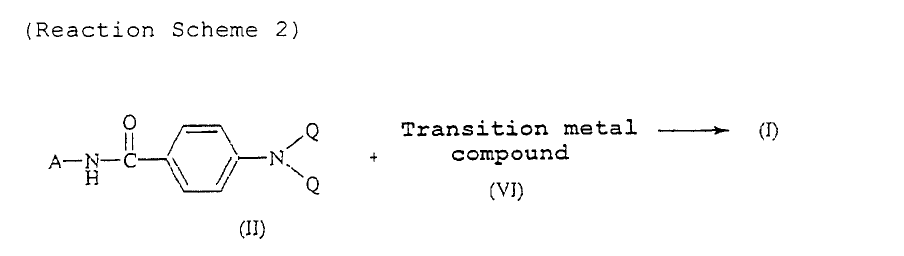 wherein A and Q have the same meanings as defined above.

To a Merrifield vessel purged with nitrogen gas, the compound (II) obtained in the reaction scheme 1 and a transition metal compound (VI) are added. The resulting mixture is shaken in an organic solvent for 30 minutes to 3 hours to afford the solid-supported transition metal complex catalyst (I) according to the present invention.

The transition metal compound (VI) can be a conjugate between copper, palladium, nickel, cobalt, rhodium or platinum and a halogen, acetic acid, trifluoroacetic acid, trifluoromethanesulfonic acid or tetrafluoroboric acid; or an allylmetal halogen dimer. As such a conjugate or dimer, one available on the market is usable. No particular limitation is imposed on the organic solvent insofar as it can dissolve the transition metal compound (VI). Examples of the organic solvent can include dichloromethane, chloroform, acetone, acetonitrile, dimethylformamide, dimethylsulfoxide, and water, with dichloromethane, acetonitrile and chloroform being preferred.

On the other hand, the solid-supported transition metal catalyst according to the present invention can be produced, for example, by heating the solid-supported transition metal complex catalyst (I), which has been obtained above in the reaction scheme 2, in the presence of an alcohol. As the alcohol, benzyl alcohol or the like can be used, and the heating can be conducted preferably at 30 to 100°C for 1 to 48 hours.

In the solid-supported transition metal catalyst obtained as described above, the transition metal is supported as a metal in any one of such compounds (II).

### Examples

The present invention will next be described further on the basis of Examples. It should, however, be borne in mind that the present invention shall not be limited to the following Examples.

### Example 1 (Production of Compound (II))

### (Step 1)

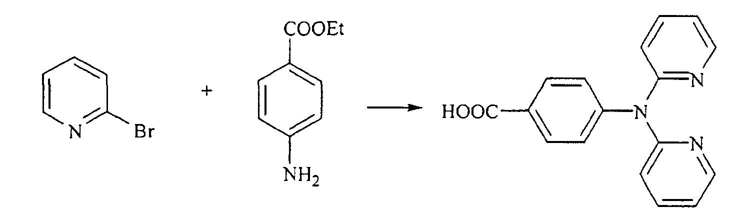

Under argon atmosphere, tris(dibenzylideneacetone)dipalladium (259 mg), rac-2,2'-bis(diphenylphosphino)-1,1'-binaphthyl (311 mg), ethyl p-aminobenzoate (1.65 g), sodium tert-butoxide (3.84 g), toluene (180 mL) and 2-bromopyridine (3.8 mL) were mixed and then stirred at 100°C for 10 hours. After the reaction mixture was poured into iced water and washed with ethyl acetate, the water layer was rendered weakly acidic with 5% hydrochloric acid and then extracted with chloroform. The extract was washed with saturated brine and then dried over sodium sulfate. The extract was filtered and concentrated, and the residue was purified by chromatography on a silica gel column (chloroform:methanol = 100:1) to yield crude crystals (1.31 g). The crude crystals were recrystallized from ethyl acetate to afford the target 4-[N,N-(2-dipyridyl)amino]benzoic acid (810 mg).
¹H-NMR (DMSO-d₆) δ:
7.01(2H,d,J=8.5Hz), 7.10(4H,m), 7.74(2H,m), 7.89(2H,d,J=8.5Hz), 8.29(2H,dd,J=2.0Hz,5.0Hz), 12.6(1H,br).

### (Step 2)

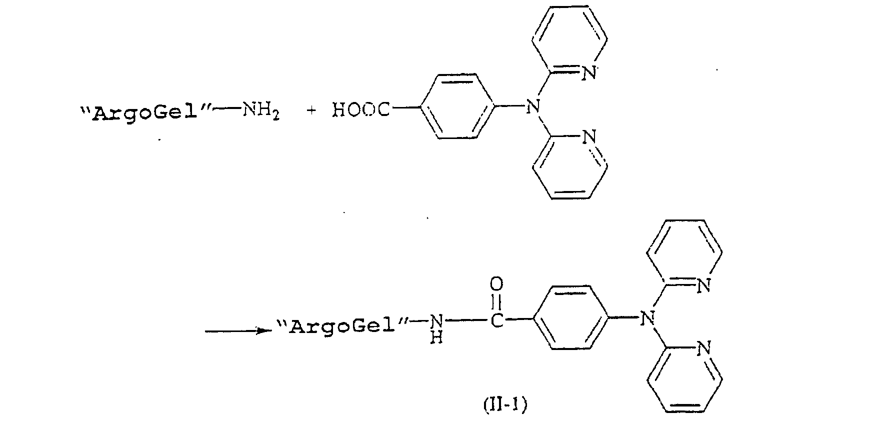

To a Merrifield vessel purged with nitrogen gas, "ArgoGel"-NH₂ (3.0 g, 1.2 mmol), 4-[N,N-(2-dipyridyl)amino]benzoic acid (524 mg, 1.8 mmol) obtained in the step 1, 1-ethyl-3-(3-dimethylaminopropyl)carbodiimide hydrochloride (460 mg, 2.4 mmol), 1-hydroxybenzotriazole (405 mg, 3.0 mmol) and dimethylformamide (60 mL) were added, followed by shaking for 18 hours. A Kaiser test was conducted with a Kaiser test reagent (product of Kokusan Chemical Co., Ltd.) to confirm an end of the reaction. Dimethylformamide (60 mL) was added, and the resulting mixture was shaken for 5 minutes, and that operation was repeated 5 times. Further, the mixture was shaken for 5 minutes in dichloromethane (60 mL), and that operation was repeated five times to wash the reaction mixture. The reaction mixture was then dried under reduced pressure to afford the target compound (II-1) (quantitative).
¹³ C-NMR (SR-MAS, CDCl₃) δ:
117.2, 118.5, 125.4, 128.2, 130.5, 137.4, 147.4, 148.3, 157.6, 166.4.

### Example 2

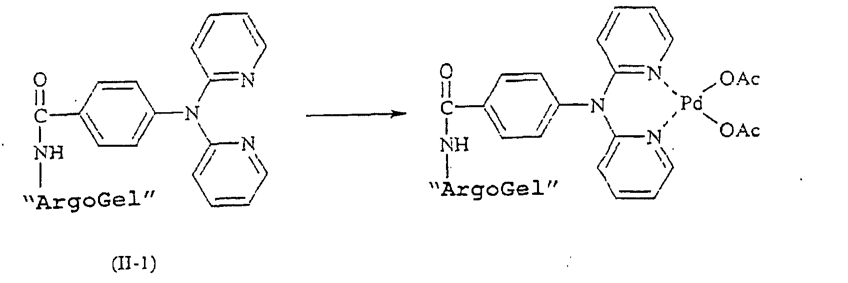

In a Merrifield vessel purged with nitrogen gas, the compound (II-1) (1.0 g) obtained in Example 1, palladium acetate (89.1 mg) and dichloromethane (20 mL) were placed, followed by shaking for 1 hour. Dichloromethane (20 mL) was added, followed by shaking for 5 minutes, and that operation was repeated 5 times to wash the reaction mixture. The reaction mixture was then dried under reduced pressure to afford the target solid-supported palladium complex catalyst (quantitative).
¹³C-NMR (SR-MAS, CDCl₃) δ:
23.0, 116.4, 117.2, 120.1, 129.7, 135.7, 140.0, 142.1, 150.3, 150.7, 165.5, 177.5.

### Example 3

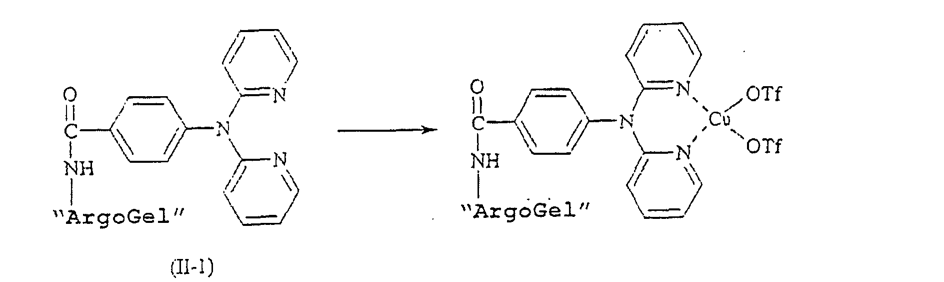

In a Merrifield vessel purged with nitrogen gas, the compound (II-1) (388 mg) obtained in Example 1, copper trifluoromethanesulfonate (55.7 mg) and acetonitrile (10 mL) were placed, followed by shaking for 1 hour. Acetonitrile (10 mL) was added, and the resulting mixture was shaken for 5 minutes. That operation was repeated 5 times. Further, the mixture was shaken for 5 minutes in dichloromethane (10 mL), and that operation was repeated 5 times to wash the reaction mixture. The reaction mixture was then concentrated under reduced pressure to afford the target solid-supported copper complex catalyst (quantitative).

### Example 4

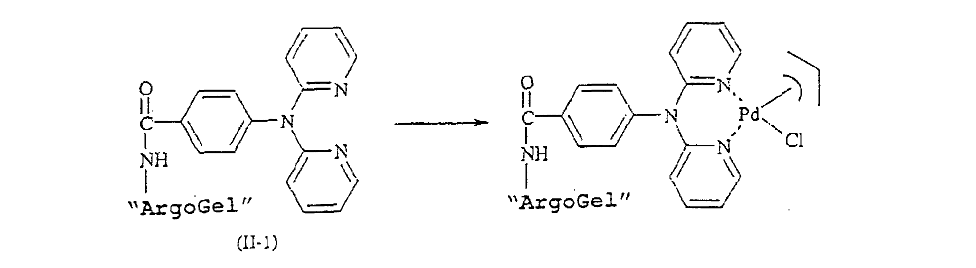

In a Merrifield vessel purged with nitrogen gas, the compound (II-1) (120 mg) obtained in Example 1, allylpalladium chloride dimer (8.7 mg) and dichloromethane (2.5 mL) were placed, followed by shaking for 1 hour. Dichloromethane (2.5 mL) was added, and the resulting mixture was shaken for 5 minutes, and that operation was repeated 5 times to wash the reaction mixture. The reaction mixture was then dried under reduced pressure to afford the target solid-supported palladium complex catalyst (quantitative). Incidentally, allylpalladium chloride dimer has the following structural formula:

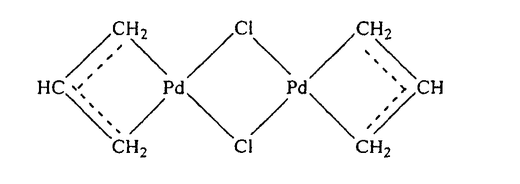

### Example 5

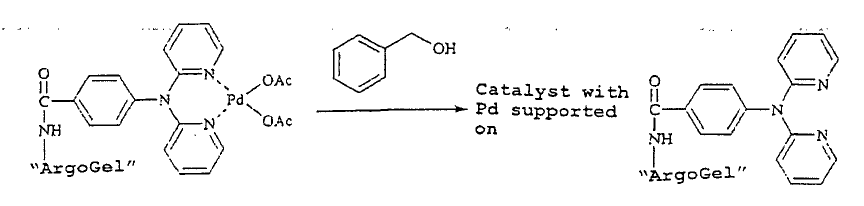

In a pear-shaped flask purged with nitrogen gas, the solid-supported palladium complex catalyst (1.0 g, 0.312 mmol) obtained in Example 2, benzyl alcohol (0.32 mL, 3.12 mmol) and water (10 mL) were placed, followed by stirring for 12 hours under refluxed heating. The reaction mixture was washed 5 times each for 5 minutes with water (20 mL), and then washed 5 times each for 5 minutes with acetone (20 mL). The reaction mixture was dried under reduced pressure to afford the target product (solid-supported palladium catalyst) as a black substance. Based on the electron micrographs shown in FIGS. 1 to 4, palladium was confirmed to be supported as a metal in the catalyst so obtained.
¹³C-NMR (SR-MAS, CDCl₃) δ:
117.2, 118.5, 125.4, 128.2, 130.5, 137.4, 147.4, 148.3, 157.6, 166.4.

### Test 1 (CO Insertion Reaction)

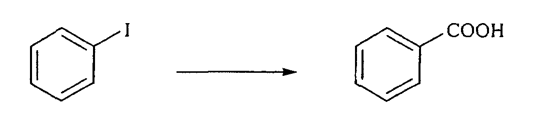

In a Merrifield vessel, the solid-supported palladium complex catalyst (140 mg) obtained in Example 4, iodobenzene (53.4 µL), potassium carbonate (59.2 mg) and water (3 mL) were placed, followed by shaking for 12 hours under carbon monoxide atmosphere. The catalyst was washed three times with a saturated aqueous solution of sodium bicarbonate (10 mL). The washings were combined together, to which 5% hydrochloric acid was added for acidification, followed by extraction with ethyl acetate. The organic layer was washed with saturated brine, dried over sodium sulfate, filtered, and then concentrated to afford benzoic acid (40 mg).

### Test 2 (Suzuki-Miyaura Reaction)

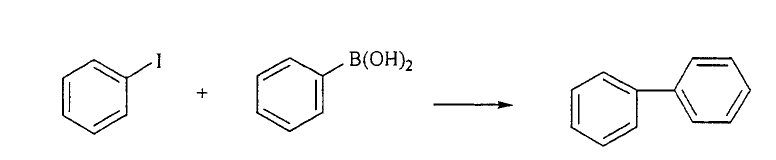

In a Merrifield vessel, the solid-supported palladium complex catalyst (137 mg) obtained in Example 4, iodobenzene (26 µL), phenylboronic acid (31.1 mg) and a 1.5 M aqueous potassium hydroxide solution (2 mL) were placed, followed by shaking for 24 hours under nitrogen gas atmosphere. The catalyst was washed three times with chloroform (10 mL). The washings were combined together, dried over sodium sulfate, filtered, and then concentrated. The residue was purified by chromatography on a silica gel column (pentane) to afford biphenyl (32 mg).

### Test 3 (Allylic Substitution Reaction)

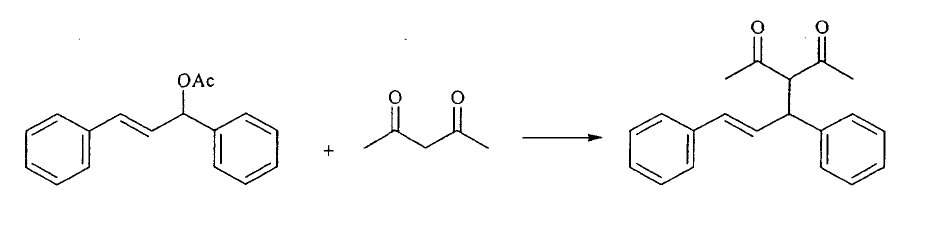

The solid-supported palladium complex catalyst (100 mg) obtained in Example 4, 1,3-diphenyl-2-propenyl acetate (85.3 mg), acetylacetone (52.1 µL), potassium carbonate (210 mg) and water (2.5 mL) were mixed, followed by stirring at 50°C for 20 hours under argon atmosphere. The catalyst was washed three times with chloroform (5 mL). The washings were combined together, dried over sodium sulfate decahydrate, filtered, and then concentrated. The residue was purified by chromatography on a silica gel column (hexane:ethyl acetate = 10:1) to afford the target compound (15 mg).

### Test 4 (Allylic Oxidation Reaction)

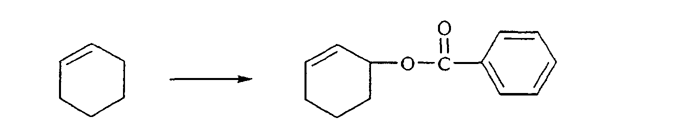

The solid-supported copper complex catalyst (46 mg) obtained in Example 3, cyclohexene (130 µL), tert-butyl perbenzoate (61 µL) and acetonitrile (1 mL) were mixed, followed by stirring at 50°C for 60 hours under argon atmosphere. The catalyst was washed three times with chloroform (5 mL). The washings were combined together, dried over sodium sulfate, filtered, and then concentrated. The residue was purified by chromatography on a silica gel column (hexane:ethyl acetate = 20:1) to afford the target compound (32 mg).

### Test 5 (Wacker Oxidation Reaction)

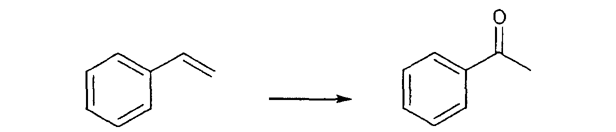

The solid-supported palladium complex catalyst (140 mg) obtained in Example 2, styrene (53.5 µL), cupric chloride (62.8 mg) and water (2 mL) were mixed, followed by heating under reflux for 60 hours under oxygen atmosphere. The catalyst was washed three times with chloroform (5 mL). The washings were combined together, dried over sodium sulfate, filtered, and then concentrated. The residue was purified by chromatography on a silica gel column (chloroform:methanol = 30:1) to afford the target compound (17 mg).

### Test 6 (Air Oxidation Reaction)

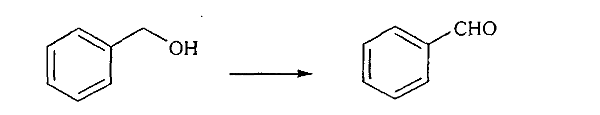

The solid-supported palladium complex catalyst (38 mg) obtained in Example 2, benzyl alcohol (13.1 µL) and water (1 mL) were mixed, followed by heating under reflux for 1 hour under oxygen atmosphere. The catalyst was washed three times with chloroform (5 mL). The washings were measured by GC/MS, and production of the target compound was confirmed (yield: 85.4%).

### Test 7 (Air Oxidation Reaction)

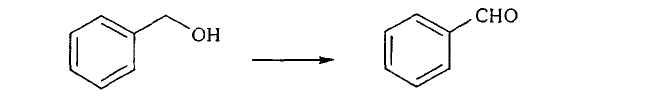

The solid-supported palladium complex catalyst recovered after the air oxidation reaction in Test 6 was dried under reduced pressure, and 30 mg of the thus-dried catalyst were mixed with benzyl alcohol (10.4 µL) and water (1 mL), followed by heating under reflux for 1 hour under oxygen atmosphere. The catalyst was washed three times with chloroform (5 mL). The washings were measured by GC/MS, and production of the target compound was confirmed (yield: 83.0%).

### Test 8 (Air Oxidation Reaction)

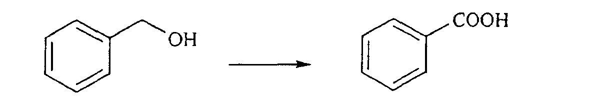

The solid-supported palladium complex catalyst (65 mg) obtained in Example 2, sodium acetate (17.8 mg), benzyl alcohol (22.5 µL) and water (1.5 mL) were mixed, followed by heating under reflux for 60 hours under oxygen atmosphere. The catalyst was washed three times with water (5 mL). The washings were rendered acidic with 5% hydrochloric acid. Further, the catalyst was washed three times with chloroform (5 mL). The washings were combined together and then subjected to extraction with chloroform. When the extract was measured by GC/MS, production of the target compound was confirmed (yield: 94.6%).

### Test 9 (Air Oxidation Reaction)

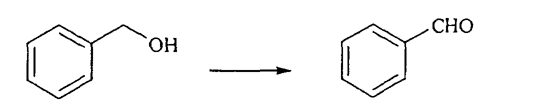

The solid-supported palladium catalyst (11.5 mg, 3.91 µmol) obtained in Example 5, benzyl alcohol (40.5 µL, 0.391 mmol) and water (0.5 mL) were mixed, followed by heating under reflux for 1.5 hours under oxygen atmosphere. The catalyst was washed three times with acetone (5 mL). By GC/MS, production of the target compound was confirmed (yield: 97%).

### Test 10 (Air Oxidation Reaction)

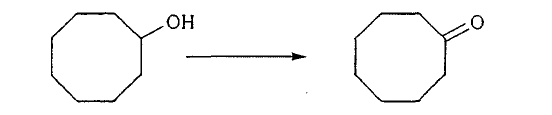

The solid-supported palladium catalyst (300 mg, 97 µmol) obtained in Example 5, cyclooctanol (64 µL, 0.49 mmol) and water (2.6 mL) were mixed, followed by heating under reflux for 20 hours under oxygen atmosphere. The catalyst was washed three times with acetone (5 mL). By GC/MS, production of the target compound was confirmed (yield: 88%).

### Test 11 (Air Oxidation Reaction)

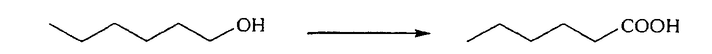

The solid-supported palladium catalyst (106 mg, 39 µmol) obtained in Example 5, 1-hexanol (22 µL, 0.17 mmol), potassium carbonate (24 mg, 0.17 mmol) and water (0.9 mL) were mixed, followed by heating under reflux for 40 hours under oxygen atmosphere. The catalyst was washed three times with a saturated aqueous sodium bicarbonate solution (5 mL). The combine filtrate was acidified with 5% hydrochloric acid, and then extracted with diethyl ether. The extract was dried and concentrated to afford the target compound (20 mg, yield: 98%).

### Industrial Applicability

Solid-supported transition metal complex catalysts and solid-supported transition metal catalysts according to the present invention are excellent catalysts which can be used for a wide variety of reactions, allow to conduct the reactions in water systems, exhibit sufficient catalytic functions even under oxygen atmosphere, and moreover, can be recovered and reused. Owing to these characteristic features, they are effective especially for combinatorial chemistry synthesis.

## Claims

1. A solid-supported transition metal complex catalyst represented by the following formula (I): 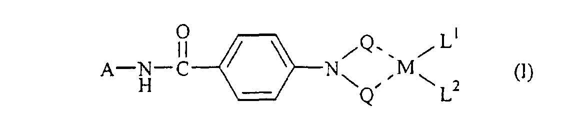 wherein A represents a polystyrene-polyethylene glycol copolymer resin, Q represents a heterocycle which may be substituted by one or more lower alkyl groups, lower alkoxy groups or halogen atoms, L¹ and L² may be the same or different and each represents a halogen atom or an acetoxy group, trifluoroacetoxy group, trifluoromethanesulfonyl group, tetrafluoroborate group or π-allyl group, and M represents copper, palladium, nickel, cobalt, rhodium or platinum.

2. A solid-supported transition metal complex catalyst according to claim 1, wherein M is copper or palladium.

3. A solid-supported transition metal complex catalyst according to claim 1 or 2, wherein said heterocycle is pyridine.

4. A solid-supported transition metal complex catalyst according to claim 1, wherein Q is pyridine, L¹ and L² may be the same or different and each represents a halogen atom or an acetoxy group, trifluoroacetoxy group or π-allyl group, and M represents copper or palladium.

5. A solid-supported transition metal catalyst comprising a compound, which is represented by the following formula (II): 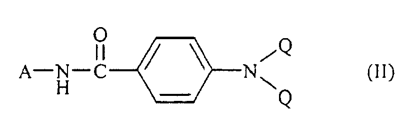 wherein A represents a polystyrene-polyethylene glycol copolymer resin and Q represents a heterocycle which may be substituted by one or more lower alkyl groups, lower alkoxy groups or halogen atoms, and a transition metal selected from copper, palladium, nickel, cobalt, rhodium or platinum and supported on said compound.

6. A solid-supported transition metal catalyst according to claim 5, wherein said transition metal is copper or palladium.

7. A solid-supported transition metal catalyst according to claim 5 or 6, wherein said heterocycle is pyridine.

8. A compound, which is represented by the following formula (II): 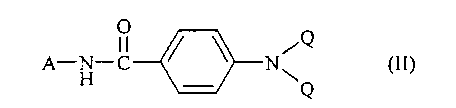 wherein A represents a polystyrene-polyethylene glycol copolymer resin, and Q represents a heterocycle which may be substituted by one or more lower alkyl groups, lower alkoxy groups or halogen atoms.
